# EUROPEAN PATENT SPECIFICATION

(11) **EP 4 436 514 B1**
(45) Date of publication and mention of the grant of the patent: **04.02.2026**
(21) Application number: 22817143.5
(22) Date of filing: 09.11.2022
(51) Int. Cl.: A61C 17/02, A61C 17/22, A46B 15/00, A61B 5/00, A61C 19/04

(54) **A SYSTEM FOR GUM ANALYSIS**
SYSTEM ZUR ZAHNFLEISCHANALYSE
SYSTÈME D'ANALYSE DE LA GENCIVE

(30) Priority: 22.11.2021 US 202163281794 P; 28.04.2022 EP 22170432
(43) Date of publication of application: 02.10.2024
(73) Proprietor: Koninklijke Philips N.V., 5656 AG Eindhoven (NL)
(72) Inventor: JOHNSON, Mark, Thomas, 5656 AG Eindhoven (NL); BRANDAO SILVA, Priscilla, 5656 AG Eindhoven (NL); KOOIJMAN, Gerben, 5656 AG Eindhoven (NL); RONDA, Cornelis, Reinder, 5656 AG Eindhoven (NL); RMAILE, Amir, Hussein, 5656 AG Eindhoven (NL)
(74) Representative: Philips Intellectual Property & Standards
(86) International application number: PCT/EP2022/081201
(87) International publication number: WO 2023/088738

(56) References cited:
- EP-A1- 3 725 263
- EP-A1- 3 725 264
- US-A1- 2011 143 310

## Description

### FIELD OF THE INVENTION

The present invention relates to systems and computer programs for gum health analysis.

### BACKGROUND OF THE INVENTION

The identification of gum recession is one of the elements of an examination by a dentist. Gum recession develops slowly and if left untreated can result in serious conditions such as periodontitis. Periodontitis is a chronic gum disease which has been reported as affecting approximately half of the adult US population, yet more than half of those affected are not diagnosed as such by their dentist. The key reason is that the screening process is time consuming and painful and can only be done by a dental practitioner.

It would therefore be desirable to provide a system able to detect gum recession as efficiently as possible, and in particular to reduce the discomfort to a patient of a prolonged examination.

It is known to incorporate sensing functionality into an oral care device, such as a toothbrush or flossing system. For example, it is known to use pressure sensing relating to a delivered fluid flow, to detect a transition from the hard tooth to softer gum tissue (and optionally also to distinguish between the tooth material and plaque). A pressure change results from the difference in flow resistance caused by the different characteristics of the different surfaces.

Such pressure sensing can for example differentiate between the teeth and gums, and also the interdental spaces.

An oral treatment device such as a toothbrush or flossing head is for example known which incorporates a fluid delivery tube through which air is pumped to an output nozzle. An interdental space can for example be detected based on a pressure sensor detecting a pressure drop or a flow rate increase in the fluid delivery tube. The flow of fluid from the fluid delivery tube is more obstructed when the nozzle is in contact with a tooth relative to when the nozzle is away from said contact. The flow of fluid from the fluid delivery tube is most obstructed when the nozzle is in contact with soft tissue such as gum tissue. This enables detection of more soft tissue, or lack of tissue such as an interdental space or indeed hard objects like teeth.

Detection of gum recession remains challenging because it proceeds slowly.

Document EP3725264A1 shows an oral cleaning system combining brushing and interdental liquid cleaning, the system induces automatic jetting at the interdental space.

Document US2011143310A1 shows a tooth surface treatment device with sensing means for controlling ejection of a fluid jet against a surface and treating the surface based on the sensed condition.

### SUMMARY OF THE INVENTION

The invention is defined by the claims.

According to examples in accordance with an aspect of the invention, there is provided a system for monitoring gum recession, comprising:
a fluid delivery tube with a proximal end configured to receive a fluid and a distal end with a nozzle for application to the teeth and gums, the nozzle having a nozzle opening;
a pump configured to pump the fluid through the fluid delivery tube;
a sensor for sensing a pressure or flow rate in the fluid delivery tube; and
a controller configured to:
   receive a pressure or flow rate profile as the nozzle is moved between a tooth surface to a gum surface; and
   determine, from characteristics of the pressure or flow rate profile at the transition between the tooth surface and the gum surface, the presence of gum recession by identifying a reduction of blockage of the nozzle opening with tissue during the movement of the nozzle across the tooth/gum boundary.

Gum recession can be conventionally be determined based on an absolute measurement of the distance from top of the tooth to the gum, and monitoring changes in this distance. However, the invention is based on the recognition that gum recession is also associated with a different gum profile (i.e. geometry). Specifically when the gum is recessed, the contour and contact angle of recessed gums to the teeth is generally higher. The more recessed the gums, generally the lower the contact angle. This change in profile can be detected when the nozzle of pressure or flow rate monitoring system is moved between the gum and tooth surfaces.

The invention is thus based on analysis of a pressure or flow signal when the nozzle moves from the tooth to gum (or vice versa), and in particular based on a different signature being present when the gum is recessed. This is a result of the contour and contact angle of recessed gums to teeth being different to when the gum is not recessed.

A magnitude indicative of a level of gum recession may also be derived from the pressure or flow rate characteristics.

The controller is adapted to determine, from characteristics of the pressure or flow rate profile, the presence of gum recession by identifying a reduction of blockage of the nozzle opening with tissue during the movement of the nozzle. Part of the nozzle (for example a side wall) may still be in contact with the tissue, but the nozzle opening may at least partially lose contact, for example because the nozzle is at a non-perpendicular angle to the gum/tooth surface. When the nozzle is at such an angle to the gum, it will leak more fluid. As the contact angle decreases further, the nozzle opening loses more contact and finally loses contact altogether.

This partial or full loss of contact arises when the gum to tooth interface has a recess so that the contact angle is less than 90 degrees (i.e. the gum surface comes down towards the tooth surface). The fluid is then pumped into a space that has been formed between the tooth surface and the gum.

The controller may be adapted to determine, from characteristics of the pressure or flow rate profile, the presence of gum recession by identifying a period of pressure reduction or flow rate increase compared to a first pressure level or flow rate during tooth contact and a second pressure level or flow rate during gum contact.

Thus, the partial or full loss of contact results in a temporary increase in flow rate or reduction in pressure, and this can be detected in the waveforms.

In one example, the sensor comprises a pressure sensor and in another example the sensor comprises a flow rate sensor.

In a first set of examples, the system may be a standalone system for monitoring gum recession. Thus the sole purpose of the system may be for analysis of gum recession.

However, in further examples, the analysis system is integrated with an oral care device, so that the analysis becomes part of the e.g. daily oral care routine of a user, without the need to perform a separate procedure to provide the analysis of gum recession.

Thus, in a first further set of examples, the invention provides a powered toothbrush device for implementing an oral care routine of tooth brushing, comprising:
a handle part;
a brushing head comprising an arrangement of bristles; and
the system defined above, wherein the nozzle is part of the brushing head, with the nozzle within or adjacent the arrangement of bristles.

Thus, the gum recession monitoring may be integrated into a powered toothbrush.

In a second further set of examples, the invention provides an oral irrigator or flossing device for implementing an oral care routine of oral irrigation, comprising:
a handle part;
an oral irrigator or flossing head;
a jetting tube between the handle part and the oral irrigator or flossing head; and
the system defined above, wherein the jetting tube functions as the fluid delivery tube, and the nozzle is the distal end of the jetting tube.

Thus, the gum recession monitoring may be integrated into an oral irrigation and/or flossing system, using the same fluid delivery system for the oral irrigation as for the gum recession analysis.

For an oral irrigator or flossing device, the controller may be adapted to control the pump in:
a first mode, for oral irrigation or flossing; and
a second mode, for gum recession sensing, wherein the flow rate for gum recession sensing is lower than the flow rate for oral irrigation or flossing.

Thus, the flow rate is matched to the function being performed. This enables a most suitable flow rate for oral irrigation or flossing to be used, which is different to a most suitable flow rate for gum recession sensing.

For an oral irrigation system, the controller may be adapted to control the pump in:
a first mode, for oral irrigation with liquid pumping; and
a second mode, for gum recession sensing, with air pumping.

This saves liquid (which needs to be supplied from a reservoir) in that it is not used for the gum recession sensing.

In all cases (oral irrigator or flossing system or toothbrush) the controller is for example adapted to receive the pressure or flow rate profile as the nozzle is moved during the oral care routine. Thus, the gum recession analysis can take place as part of the oral care routine of the user of the system. The user may, for example, be instructed to make certain movements (e.g. up and down between the tooth and gum) as part of the oral care routine.

The device may comprise a motion or position sensor for detecting when, during an oral care routine, there is a movement of the nozzle between a tooth surface to a gum surface.

Thus, the movements which are suitable for performing a gum recession analysis may be detected. The motion or position sensor for example comprises an inertial measurement unit comprising an accelerometer and gyroscope. A a 6-axis measurement unit may be used so that positions and movements in 3D space can be tracked.

The controller may be adapted to use the motion or position sensor output to detect when the nozzle is moved away from the tooth and gum surface rather than along the tooth and gum surface. In this way, a loss of contact caused by movement of the nozzle can be distinguished from a loss of contact caused by gum recession.

The controller may be adapted to build a model of the locations of the teeth and gum boundaries from the motion and position sensing and from the gum recession sensing.

The system of the invention is also used in a method for monitoring gum recession, comprising:
controlling a pump to pump a fluid through a fluid delivery tube while a user applies a nozzle at the end of the fluid delivery tube to the teeth and gums;
receiving a sensor signal sensing a pressure or flow rate in the fluid delivery tube; and
determining from characteristics of the pressure or flow rate profile, at the transition between the tooth surface and the gum surface, the presence of gum recession by identifying a reduction of blockage of the nozzle opening with tissue during the movement of the nozzle across the tooth/gum boundary.

The invention also provides a computer program comprising computer program code which is adapted, when said program is run on a controller of the system for monitoring gum recession as defined above, to implement the method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a schematic representation of a system only for analyzing gum recession;
Figure 2 show a tooth and healthy gums and shows the sensor output signal when the nozzle of the system of Figure 1 is moved across the tooth/gum boundary;
Figure 3 show a tooth and recessed gums and shows the sensor output signal when the nozzle of the system of Figure 1 is moved across the tooth/gum boundary;
Figure 4 shows a schematic of the head section of a toothbrush incorporating the analysis system;
Figure 5 shows a schematic representation of the overall toothbrush combined with an oral irrigator and the analysis system; and
Figure 6 shows a schematic representation of an air flossing and oral irrigation system incorporating the analysis system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and computer programs of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for monitoring gum recession. A nozzle is for application to the teeth and gums, and a fluid is pumped to a nozzle opening through a delivery tube. A sensor is provided for sensing a pressure or flow rate so that a pressure profile or flow rate profile is derived as the nozzle is moved between a tooth surface and a gum surface, From characteristics of the pressure or flow rate profile at the transition between the tooth surface and the gum surface, the presence of gum recession can be detected.

The invention may be implemented as a device only for gum analysis or as part of a toothbrush or an oral irrigator or a flossing system, or part of a system combining two or more of tooth brushing, oral irrigation (using a liquid) and flossing (using liquid and air).

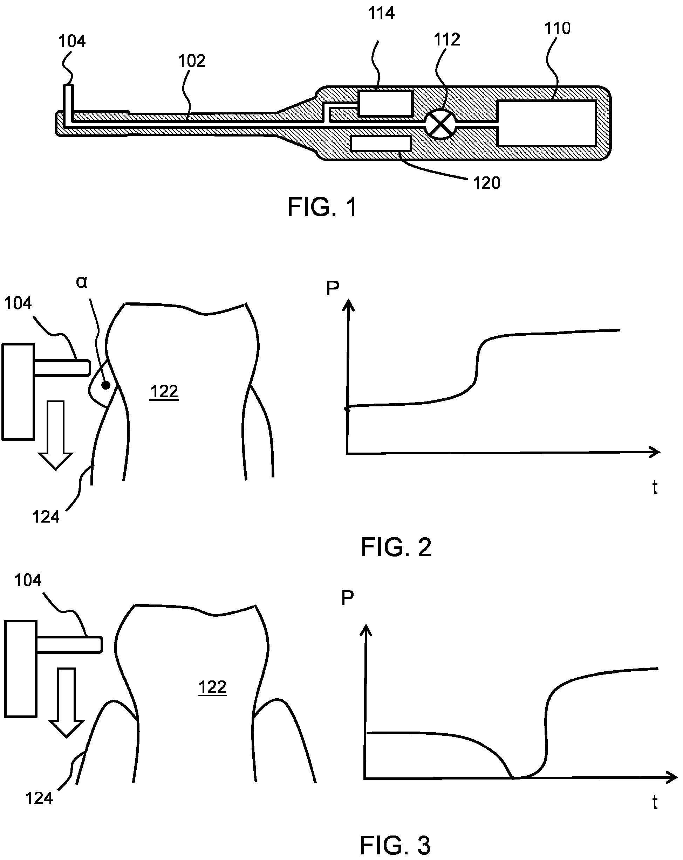

Figure 1 shows a schematic representation of a system only for analyzing gum recession.

The system comprises a nozzle 104 for application to the teeth and gums. The nozzle is the distal end of a fluid delivery tube 102 and has a nozzle opening. The proximal end is configured to receive a fluid from a reservoir 110 or it may use ambient air as a delivery fluid.

A pump 112 is configured to pump the fluid through the fluid delivery tube 102 to the nozzle 104 so that it exits through the nozzle opening. A sensor 114 is provided for sensing a pressure in the fluid delivery tube 102 or a flow rate along the fluid delivery tube 102.

A controller 120 receives the pressure or flow rate profile as the nozzle is moved between a tooth surface and a gum surface. As explained further below, from characteristics of the pressure profile or flow rate profile, in particular at the transition between the tooth surface and the gum surface, the presence of gum recession can be detected and preferably also quantified.

In one example, the sensor 114 is a pressure sensor. However, the sensor 114 could also be a flow sensor or a combined sensor which can measure flow rate and pressure inside the fluid delivery tube.

A decrease in pressure in the fluid delivery tube 102 can be used to indicate that the nozzle has reached a reduced flow resistance, which may for example be the result of the nozzle facing a cavity area, such as interdental space or a cavity formed by gum recession, as explained below.

The pump 112 is used to drive a fluid through the fluid delivery tube 102 from the fluid reservoir 110. The pressure sensor 114 is used to determine directly, or indirectly, the flow of fluid and/or the pressure of the fluid through the fluid delivery tube 102.

In operation of the device of Figure 1, the user (e.g. an oral health professional) will hold the nozzle to the teeth or gum surface.

Figure 2 shows the nozzle 104 against the side of a tooth 122 with healthy gums 124, and also shows the pressure P over time t as the nozzle is moved from the top of the tooth to the bottom of the gum. The nozzle is thus moved across the tooth/gum boundary.

In the case of a normal gum pathology, there is no pocket at the boundary between the gum and the tooth, so the angle α between the gum and the teeth approaches or exceeds 90 degrees. As a consequence, as the nozzle 104 moves from the tooth to the gum (or vice versa), the nozzle opening remains in contact with the surface at all times. As a consequence there is always an excess pressure which increases from the hard tooth surface to the softer gum surface, because the nozzle opening is better blocked by the softer gum which therefore presents an increased flow resistance.

The resulting pressure signature shown in Figure 2 is a steady monotonic increase of pressure across the boundary from a first pressure level (blockage by the teeth) to a second pressure level (blockage by the gums).

Figure 3 shows the nozzle against the side of a tooth 122 with gums 124 with gum recession, and again shows the pressure P over time t as the nozzle is moved from the top of the tooth to the bottom of the gum. The nozzle is thus again moved across the tooth/gum boundary.

In the case of recessed gums, a clear pocket starts to form and the angle between the gum and the teeth reduces and may be less than 90 degrees, as shown. As a consequence, as the nozzle moves from the tooth to the gum (or vice versa), the presence of the developing pocket and the associated lower angle causes the nozzle opening to lose contact with the surface as the boundary is crossed. There is therefore a drop in the pressure, as the nozzle opening is not blocked, to a lower value than that which results from contact with either the hard tooth surface or the softer gum surface.

The resulting pressure signature shown in Figure 3 has a short reduction of pressure across the boundary between the first pressure (blockage by the teeth) and the second pressure (blockage by the gums). The drop in pressure is a signature of gum recession.

Of course, there is also an intermediate case where the nozzle opening is partly blocked. A partially blocked nozzle opening is for example indicative of a beginning of gum recession. The ability to detecting gum recession early is a significant benefit of the system.

Thus, as the contact angle decreases, the nozzle opening is no longer fully blocked by the tissue. Part of the nozzle may still be in contact (for example a side wall) but the nozzle opening has (partially) lost contact i.e. it will be at an angle to the gum and therefore leaking more fluid.

The longer (in time duration) of the pressure drop (for a given speed of movement of the nozzle), the more likely that the recession is advanced. Thus, analysis of the characteristics of the pressure profile also enable a level of gum recession to be quantified.

The controller 120 of the system can thereby determine, from characteristics of the pressure or flow rate profile, the presence of gum recession by identifying a loss or partial loss of contact of the nozzle with tissue during the movement of the nozzle. In particular, the controller can identify a period of pressure reduction or flow increase compared to a first pressure level or flow rate during tooth contact and a second pressure level or flow rate during gum contact.

Thus, the profiles shown in Figures 2 and 3 can be monitored for localized regions of reduced pressure (or increased flow rate), by setting thresholds relating both to the pressure (or flow rate) levels and relating to timing characteristics, so that signal characteristics which are caused by other effects can be filtered out.

Many different signal processing approaches may be used to determine features indicative of the presence and severity of the gum recession. For example, a nonmonotonic waveform may be identified by a change in sign of the first derivative of the waveform.

The waveform analysis is for example in the time domain. It may use first or second derivatives as well as the bare signal. It may also take into account the position and speed and direction of the nozzle as detected by motion sensing, as discussed further below. For example, the shape of the waveforms may be converted into a pressure versus position waveform, so that the waveform analysis may then be considered to be in the position domain. In particular, the shape of the pressure-time waveform will depend on the speed of movement of the nozzle over the gum/tooth boundary, whereas a pressure-position waveform more accurately conveys gum shape information.

While Figures 2 and 3 show pressure signatures, if it is preferred to use a flow rate sensor (flow meter) the graphs will be inverted, with flow being lowest on the gums, intermediate on the teeth and highest when the nozzle loses contact at the recessed gum pocket. The sensor could also be a combined sensor which can measure flow rate and pressure inside the fluid delivery tube.

The examples above show a system specifically for detecting gum recession. The system may however be combined with an oral care device which has the main purpose of performing an oral care routine.

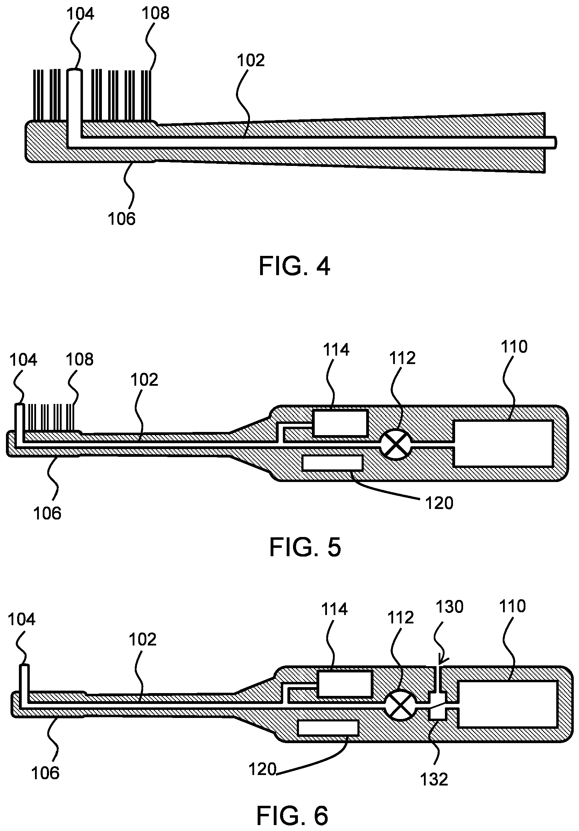

Figure 4 shows schematically the head section 106 of a toothbrush. The fluid delivery tube 102 with the nozzle 104 at the distal end is incorporated into the toothbrush head 106. The nozzle is positioned at a suitable position between or close to the bristle tufts of the toothbrush head. The nozzle 104 protrudes from the head section 106 in the same (or largely the same) direction as the bristles 108 of the toothbrush.

The fluid delivery tube 102 and the nozzle can be used for the gum analysis as described above.

In addition, the fluid delivery tube 102 and the nozzle 104 may also be used for oral irrigation or for flossing.

Figure 5 shows the head of Figure 4 incorporated into a combined oral irrigation and tooth brushing system.

In this example, the fluid reservoir 110, pump 112 and sensor 114 are part of the toothbrush. However, these components may be separate to the toothbrush and connected by the fluid delivery tube 102.

In operation, the pressure or flow is monitored as discussed above and gum recession is identified from the same signal traces. However, the gum recession analysis may now occur during a regular tooth brushing (or oral irrigation) session. The traces are for example captured as the user moves the toothbrush around the mouth and the brush head moves across the tooth/gum boundary.

A relatively low flow rate of the liquid may be used for sensing gum recession and a relatively high rate may be used for the oral irrigation cleaning function. In this manner, the jetting liquid is more efficiently used and the user does not have to deal with excessive amounts of liquid in their mouth.

In an example, positioning capability such as an inertial monitoring unit with one or more accelerometers (such as a 3-axis accelerometer) and one or more gyroscopes (such as a 3-axis gyroscope) may be used in particular to determine when a vertical movement is made, so that the nozzle has most likely crossed a gum/tooth boundary. Any suitable absolute or relative positioning capability, as known in the prior art, may be used. For example, position can be obtained by double integration of the accelerometer signals of an inertia monitoring unit after removal of the gravity component (which itself is derived from both the gyroscope and accelerometer signals).

For measuring displacements over a relatively short time interval, large accuracy is not needed and low cost sensing can provide the required results.

By way of example, displacement measurement based on processing the acceleration signals may be started on detection of a drop in pressure indicative of a gum recess, as discussed above, and stopped on detection of an increase in pressure (due to moving from pocket to gum).

The displacement measurement during this change-in-pressure-event can serve two purposes:
First, tracking the direction of the displacement helps to eliminate false detections. For example, if a detected displacement is perpendicular to the tooth/gum surface, a similar pressure change may arise as a result of the nozzle being taken off the tooth and subsequently placed back on the tooth or gums, not because of crossing a pocket caused by gum recession. When the detected direction of displacement is along the tooth/gums, the pressure change is due to crossing a pocket.
Second, the detected displacement value may be an indication of the severity of the pocket. As explained above, position information enables a better interpretation of the pressure (or flow rate) signals because it enables a conversion into position value instead of time values.

Figure 5 shows the delivery of a liquid from a reservoir 110, and such liquid delivery is known for an oral irrigator.

The gum analysis of the invention may instead be based on a combined liquid and gas flow (e.g.. air). The use of liquid droplets entrained in an air flow is for example also known for an air flossing system. Thus, the invention may be combined with an air flossing system. The invention may in principle also be applied to a system which delivers only air. An air based system is for example known for plaque detection. In all of these cases, the air system may be used for the recessed gum detection.

Figure 6 shows an arrangement which can deliver air or liquid to the delivery tube, so that it can perform both an air flossing function (with liquid droplets entrained in an air stream) or a liquid oral irrigation function. The pump 112 is able to provide pumping of air by means of an air inlet 130 and a switch 132 between the fluid reservoir 110 and the air inlet 130. The switch 132 is configured to select whether the pump 112 delivers ambient air, or a liquid from the reservoir, through the fluid delivery tube 102.

Alternatively, a second fluid reservoir with air (or any gas) may be used instead of the air inlet 130. One or more air inlets 130 may also be used, to ensure that if any of the air inlets is blocked by the hand of the user, the pump can still pump air.

In this design, the gum recession analysis may use the air flow or the liquid flow. The pump may for example be controlled to provide an air flow through the fluid delivery tube 102 and the nozzle 104 for the gum recession detection, whereas a liquid burst or flow may be used for the interdental cleaning, i.e. the oral irrigation function.

In this manner, the jetting liquid is again more efficiently used. Thus, a combination of a gas flow, to perform the recessed gum analysis (and air flossing) and liquid flow for cleaning may be used.

The analysis may also be used to identify interdental areas, again for example using the air flow, so that the liquid flow can be interrupted when the nozzle is not correctly positioned for the interdental cleaning. Thus, the controller can be used to control the pump to pump air when the sensor has not detected an interdental space, and a liquid (e.g. cleaning fluid) when the pressure sensor detects a decrease in pressure in the fluid delivery tube corresponding to an interdental space.

The gum recession analysis can take place during regular irrigation, brushing or air flossing sessions. In the case of an air flossing device, the user may require extra instructions to move the nozzle across the tooth/gum boundary as this may not be the case in the usual operation mode.

The waveforms are thus captured as the user moves the device head around the mouth during their normal oral care routine and the nozzle moves across the tooth/gum boundary.

In examples having motion sensing, the device may be used to derive a 3D profile of the teeth and gums. Based on a daily oral care routine using a device with the recessed gum detection, the device can be used to detect where the gums are and where the teeth are. Based on repeated analysis over time, the profile can be iterated and improved, to obtain 3D scan-like file of the teeth and gums.

To improve the 3D profile, a guided brushing (or other oral care routine) technique may be used, whereby the user is asked to follow a certain pattern when brushing, to facilitate localization, as well as to enable estimation of the vertical dimension of the teeth. For example, by asking the user to brush from the gum upwards, three different sets of spectra will be recorded, for the gums, teeth, and free space, thereby indicating the edge or height of the tooth. The user may then be instructed to move to the next tooth and so on.

The captured sensor data may be combined with a 2D photo (e.g. by superimposing), so that any missing information from one modality (e.g. camera) is provided by the other modality (e.g. sensing).

The invention enables an early indication of gum recession, in particular because the sensing can be made as a daily measurement of the gum/tooth boundary during a daily oral care routine, and the initial stages of gum recession can be detected based on small changes in the blockage of the nozzle opening, as discussed above.

Federated data from many different users can also be used and interpreted and fed back into each individual device by updates.

Variations to the disclosed embodiments can be understood and effected by those skilled in the art in practicing the claimed invention, from a study of the drawings and the disclosure, the scope of the invention being defined by the wording of the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

The skilled person would be readily capable of developing a controller for carrying out any herein described method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for monitoring gum recession, comprising:
a fluid delivery tube (102) with a proximal end configured to receive a fluid and a distal end with a nozzle (104) for application to the teeth and gums, the nozzle having a nozzle opening;
a pump (112) configured to pump the fluid through the fluid delivery tube;
a sensor (114) for sensing a pressure or flow rate in the fluid delivery tube; and
a controller (120) configured to:
receive a pressure or flow rate profile as the nozzle is moved between a tooth surface to a gum surface; and
determine, from characteristics of the pressure or flow rate profile at the transition between the tooth surface and the gum surface, the presence of gum recession by identifying a reduction of blockage of the nozzle opening with tissue during the movement of the nozzle across the tooth/gum boundary.

2. The system of claim 1, wherein the controller (120) is adapted to determine, from characteristics of the pressure or flow rate profile, the presence of gum recession by identifying a period of pressure reduction or flow increase compared to a first pressure level or flow rate during tooth contact and a second pressure level or flow rate during gum contact.

3. A powered toothbrush device for implementing an oral care routine of tooth brushing, comprising:
a handle part;
a brushing head (106) comprising an arrangement of bristles; and
the system of any one of claims 1 to 2, wherein the nozzle (104) is part of the brushing head, with the nozzle within or adjacent the arrangement of bristles.

4. An oral irrigator device for implementing an oral care routine of oral irrigation or flossing, comprising:
a handle part;
an oral irrigator or flossing head (106);
a jetting tube (102) between the handle part and the oral irrigator or flossing head; and
the system of any one of claims 1 to 2, wherein the jetting tube (102) functions as the fluid delivery tube, and the nozzle is the distal end of the jetting tube.

5. The device of claim 4, wherein the controller (120) is adapted to control the pump in:
a first mode, for oral irrigation or flossing ; and
a second mode, for gum recession sensing, wherein the flow rate for gum recession sensing is lower than the flow rate for oral irrigation or flossing.

6. The device of claim 4, wherein the controller is adapted to control the pump in:
a first mode, for oral irrigation with liquid pumping; and
a second mode, for gum recession sensing, with gas pumping.

7. The device of any one of claims 3 to 6, wherein the controller (120) is adapted to receive the pressure or flow rate profile as the nozzle is moved during the oral care routine.

8. The device of any one of claims 3 to 7, comprising a motion or position sensor for detecting when, during an oral care routine, there is a movement of the nozzle between a tooth surface to a gum surface.

9. The device of claim 8, wherein the motion or position sensor comprises an inertial measurement unit comprising an accelerometer and gyroscope.

10. The device of any claim 8 or 9, wherein the controller (120) is adapted to use the motion or position sensor output to detect when the nozzle is moved away from the tooth and gum surface rather than along the tooth and gum surface.

11. The device of any one of claims 8 to 10, wherein the controller (120) is adapted to build a model of the locations of the teeth and gum boundaries from the motion and position sensing and from the gum recession sensing.

12. A computer program comprising computer program code which is adapted, when said program is run on a controller of the system for monitoring gum recession of any one of claims 1 to 2, to implement a method for monitoring gum recession, the method comprising:
controlling a pump to pump a fluid through a fluid delivery tube while a user applies a nozzle at the end of the fluid delivery tube, with a nozzle opening, to the teeth and gums;
receiving a sensor signal sensing a pressure or flow rate in the fluid delivery tube; and
determining from characteristics of the pressure or flow rate profile, at the transition between the tooth surface and the gum surface, the presence of gum recession by identifying a reduction of blockage of the nozzle opening with tissue during the movement of the nozzle across the tooth/gum boundary.

## Patentansprüche

1. System zum Überwachen von Zahnfleischschwund, umfassend:
einen Flüssigkeitsabgabeschlauch (102) mit einem proximalen Ende, das konfiguriert ist, eine Flüssigkeit aufzunehmen, und einem distalen Ende mit einer Düse (104) zur Anwendung auf die Zähne und Zahnfleisch, wobei die Düse eine Düsenöffnung aufweist;
eine Pumpe (112), die konfiguriert ist, die Flüssigkeit durch den Flüssigkeitsabgabeschlauch zu pumpen;
einen Sensor (114) zum Erfassen eines Drucks oder einer Durchflussrate im Flüssigkeitsabgabeschlauch; und eine Steuereinheit (120), die konfiguriert ist:
ein Druck- oder Durchflussratenprofil zu empfangen, wenn die Düse zwischen einer Zahnoberfläche und einer Zahnfleischoberfläche bewegt wird; und
aus den Eigenschaften des Druck- oder Durchflussratenprofils am Übergang zwischen der Zahnoberfläche und der Zahnfleischoberfläche das Vorhandensein von Zahnfleischschwund durch Identifizieren einer Verringerung der Verstopfung der Düsenöffnung durch Gewebe bei der Bewegung der Düse über die Zahn/Zahnfleischgrenze zu bestimmen.

2. System nach Anspruch 1, wobei die Steuereinheit (120) angepasst ist, aus den Eigenschaften des Druck- oder Durchflussratenprofils das Vorhandensein von Zahnfleischschwund durch Identifizieren eines Zeitraums von Druckverringerung oder Durchflusssteigerung im Vergleich zu einer ersten Druckstufe oder Durchflussrate während des Zahnkontakts und einer zweiten Druckstufe oder Durchflussrate während des Zahnfleischkontakts zu bestimmen.

3. Elektrische Zahnbürstenvorrichtung zum Implementieren einer Mundpflegeroutine des Zähneputzens, umfassend:
einen Griffteil;
ein Bürstenkopf (106), der eine Anordnung von Borsten umfasst; und
das System nach einem der Ansprüche 1 oder 2, wobei die Düse (104) Teil des Bürstenkopfes ist, mit der Düse innerhalb oder neben der Anordnung von Borsten.

4. Mundduschvorrichtung zum Implementieren einer Mundpflegeroutine des Mundduschens oder der Verwendung von Zahnseide, umfassend:
einen Griffteil;
eine Munddusche oder einen Zahnseidenkopf (106);
einen Spülschlauch (102) zwischen dem Griffteil und dem Mundduschen- oder Zahnseidenkopf; und
ein System nach einem der Ansprüche 1 bis 2, wobei der Spülschlauch (102) als Flüssigkeitsabgabeschlauch fungiert und die Düse das distale Ende des Spülschlauches ist.

5. Vorrichtung nach Anspruch 4, wobei die Steuereinheit (120) angepasst ist, die Pumpe zu steuern in:
einem ersten Modus, zum Mundduschen oder Verwenden von Zahnseide; und
einem zweiten Modus zum Erfassen von Zahnfleischschwund, wobei die Durchflussrate zum Erfassen von Zahnfleischschwund niedriger ist als die Durchflussrate zum Mundduschen oder Verwenden von Zahnseide.

6. Vorrichtung nach Anspruch 4, wobei die Steuereinheit angepasst ist, die Pumpe zu steuern in:
einem ersten Modus zum Mundduschen mit Pumpen von Flüssigkeit; und
einem zweiten Modus zum Erfassen von Zahnfleischschwund mit Pumpen von Gas.

7. Vorrichtung nach einem der Ansprüche 2 bis 6, wobei die Steuereinheit (120) angepasst ist, das Druck- oder Durchflussratenprofil zu empfangen, wenn die Düse während der Mundpflegeroutine bewegt wird.

8. Vorrichtung nach einem der Ansprüche 3 bis 7, umfassend einen Bewegungs- oder Positionssensor zum Erkennen, wann während einer Mundpflegeroutine eine Bewegung der Düse zwischen einer Zahnoberfläche zu einer Zahnfleischoberfläche stattfindet.

9. Vorrichtung nach Anspruch 8, wobei der Bewegungs- oder Positionssensor eine Trägheitsmesseinheit umfasst, die einen Beschleunigungsmesser und ein Gyroskop umfasst.

10. Vorrichtung nach Anspruch 8 oder 9, wobei die Steuereinheit (120) angepasst ist, den Bewegungs- oder Positionssensorausgang zu verwenden, um zu erkennen, wann die Düse von der Zahn- und Zahnfleischoberfläche weg anstatt entlang der Zahn- und Zahnfleischoberfläche bewegt wird.

11. Vorrichtung nach einem der Ansprüche 8 bis 10, wobei die Steuereinheit (120) angepasst ist, aus der Bewegungs- und Positionserfassung und aus der Erfassung des Zahnfleischschwunds ein Modell der Stellen der Zahn- und Zahnfleischgrenzen aufzubauen.

12. Computerprogramm, umfassend einen Computerprogrammcode, der angepasst ist, um, wenn das Programm auf einer Steuereinheit des Systems zum Überwachen des Zahnfleischschwunds nach einem der Ansprüche 1 oder 2 läuft, ein Verfahren zum Überwachen des Zahnfleischschwunds zu implementieren, das Verfahren umfassend:
Steuern einer Pumpe, um eine Flüssigkeit durch einen Flüssigkeitsabgabeschlauch zu pumpen, während ein Benutzer eine Düse am Ende des Flüssigkeitsabgabeschlauches mit einer Düsenöffnung an Zähne und Zahnfleisch ansetzt;
Empfangen eines Sensorsignals, das einen Druck oder eine Durchflussrate im Flüssigkeitsabgabeschlauch erfasst; und
Bestimmen aus den Eigenschaften des Druck- oder Durchflussratenprofils am Übergang zwischen der Zahnoberfläche und der Zahnfleischoberfläche das Vorhandensein von Zahnfleischschwund durch Identifizieren einer Verringerung der Verstopfung der Düsenöffnung durch Gewebe bei der Bewegung der Düse über die Zahn/Zahnfleischgrenze.

## Revendications

1. Système de surveillance de la récession gingivale, comprenant :
un tube de distribution de fluide (102) avec une extrémité proximale configurée pour recevoir un fluide et une extrémité distale avec une buse (104) pour application sur les dents et les gencives, la buse présentant une ouverture de buse ;
une pompe (112) configurée pour pomper le fluide à travers le tube de distribution de fluide ;
un capteur (114) pour détecter une pression ou un débit dans le tube de distribution de fluide ; et un dispositif de commande (120) configuré pour :
recevoir un profil de pression ou de débit lorsque la buse est déplacée entre une surface de dent et une surface de gencive ; et
déterminer, à partir de caractéristiques du profil de pression ou de débit au niveau de la transition entre la surface de dent et la surface de gencive, la présence d'une récession gingivale par l'identification d'une réduction d'obstruction de l'ouverture de buse par des tissus lors du déplacement de la buse à la limite dent/gencive.

2. Système selon la revendication 1, dans lequel le dispositif de commande (120) est adapté pour déterminer, à partir de caractéristiques du profil de pression ou de débit, la présence d'une récession gingivale par l'identification d'une période de réduction de pression ou d'augmentation de débit par rapport à un premier niveau de pression ou de débit pendant un contact dentaire et à un second niveau de pression ou de débit pendant un contact gingival.

3. Dispositif de brosse à dents électrique pour mettre en œuvre une routine de soins buccaux de brossage de dents, comprenant :
une partie manche ;
une tête de brossage (106) comprenant un agencement de poils ; et
le système selon l'une quelconque des revendications 1 et 2, dans lequel la buse (104) fait partie de la tête de brossage, la buse se trouvant à l'intérieur ou à proximité de l'agencement de poils.

4. Dispositif d'irrigation buccale pour mettre en œuvre une routine de soins buccaux d'irrigation buccale ou de passage de fil dentaire, comprenant :
une partie manche ;
un irrigateur buccal ou une tête de fil dentaire (106) ;
un tube de jet (102) entre la partie manche et l'irrigateur buccal ou la tête de fil dentaire ; et
le système selon l'une quelconque des revendications 1 et 2, dans lequel le tube de jet (102) fonctionne comme tube de distribution de fluide, et la buse est l'extrémité distale du tube de jet.

5. Dispositif selon la revendication 4, dans lequel le dispositif de commande (120) est adapté pour commander la pompe dans :
un premier mode, pour l'irrigation buccale ou le passage du fil dentaire ; et
un second mode, pour la détection de la récession gingivale, dans lequel le débit pour la détection de la récession gingivale est inférieur au débit pour l'irrigation buccale ou le passage du fil dentaire.

6. Dispositif selon la revendication 4, dans lequel le dispositif de commande est adapté pour commander la pompe dans :
un premier mode, pour l'irrigation buccale par pompage de liquide ; et
un second mode, pour la détection de la récession gingivale, par pompage de gaz.

7. Dispositif selon l'une quelconque des revendications 3 à 6, dans lequel le dispositif de commande (120) est adapté pour recevoir le profil de pression ou de débit lorsque la buse est déplacée pendant la routine de soins buccaux.

8. Dispositif selon l'une quelconque des revendications 3 à 7, comprenant un capteur de mouvement ou de position pour détecter quand, au cours d'une routine de soins buccaux, la buse est déplacée entre une surface de dent et une surface de gencive.

9. Dispositif selon la revendication 8, dans lequel le capteur de mouvement ou de position comprend une unité de mesure inertielle comprenant un accéléromètre et un gyroscope.

10. Dispositif selon l'une quelconque des revendications 8 et 9, dans lequel le dispositif de commande (120) est adapté pour utiliser la sortie du capteur de mouvement ou de position afin de détecter quand la buse est éloignée de la surface de dent et de gencive plutôt que déplacée le long de la surface de dent et de gencive.

11. Dispositif selon l'une quelconque des revendications 8 à 10, dans lequel le dispositif de commande (120) est adapté pour construire un modèle des emplacements des limites de dent et de gencive à partir de la détection de mouvement et de position et de la détection de la récession gingivale.

12. Programme informatique comprenant un code de programme informatique qui, lorsqu'il est exécuté sur un dispositif de commande du système de surveillance de la récession gingivale selon l'une quelconque des revendications 1 et 2, est adapté pour mettre en œuvre un procédé de surveillance de la récession gingivale, le procédé comprenant :
la commande d'une pompe pour faire circuler un fluide à travers un tube de distribution de fluide pendant qu'un utilisateur applique une buse à l'extrémité du tube de distribution de fluide, muni d'une ouverture, sur les dents et les gencives ;
la réception d'un signal de capteur détectant une pression ou un débit dans le tube de distribution de fluide ; et
la détermination, à partir de caractéristiques du profil de pression ou de débit, au niveau de la transition entre la surface de dent et la surface de gencive, de la présence d'une récession gingivale par l'identification d'une réduction d'obstruction de l'ouverture de buse par des tissus lors du déplacement de la buse à la limite dents/gencive.
